(19) Europäisches Patentamt
European Patent Office
Office européen des brevets

(11) EP 0 456 107 B1

(12) **EUROPÄISCHE PATENTSCHRIFT**

(45) Veröffentlichungstag und Bekanntmachung des Hinweises auf die Patenterteilung:
**17.01.1996 Patentblatt 1996/03**

(51) Int Cl.[6]: **C12P 7/02**, C12P 7/22, C12P 7/26, C12P 41/00, C12N 9/04

(21) Anmeldenummer: **91107067.0**

(22) Anmeldetag: **02.05.1991**

(54) **Enzymatisches Verfahren zur Reduktion von Oxoverbindungen, insbesondere Acetophenon und dafür geeignetes Enzym**

Process for the enzymatic reduction of oxo-compounds, especially acetophenone and the enzyme therefor

Procédé enzymatique de réduction de composés oxygénés, en particulier l'acétophénone et l'enzyme pour cela

(84) Benannte Vertragsstaaten:
**CH DE DK FR GB IT LI NL**

(30) Priorität: **07.05.1990 DE 4014573**

(43) Veröffentlichungstag der Anmeldung:
**13.11.1991 Patentblatt 1991/46**

(73) Patentinhaber:
**FORSCHUNGSZENTRUM JÜLICH GMBH**
**D-52425 Jülich (DE)**

(72) Erfinder: **Hummel, Werner, Dr.**
**W-5177 Titz (DE)**

(56) Entgegenhaltungen:
- **APPLIED MICROBIOLOGY & BIOTECHNOLOGY, Band 34, Nr. 1, Oktober 1990; W. HUMMEL, Seiten 15-19**
- **BIOTECHNOLOGY LETTERS, Band 12, Nr. 6, Juni 1990; W. HUMMEL, Seiten 403-408**
- **APPLIED MICROBIOLOGY & BIOTECHNOLOGY, Band 24, Nr. 2, Mai 1986; F. ARAGOZZINI et al., Seiten 175-177**
- **PATENT ABSTRACTS OF JAPAN, Band 11, Nr. 150 (C-422)(2597), 15 Mai 1987**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Gegenstand der Erfindung ist eine Zur Reduktion von Acetophenon zu praktisch enantiomeren-reinem R(+)-Phenylethanol in Gegenwart von NADPH befähigte, aus Lactobacillen isolierbare Phenylethanol-Dehydrogenase mit folgendem weiteren Reaktionsspektrum

- rasche Desaktivierung durch EDTA, aber geringer Einfluß von 2,2-Dipyridin; 1,10-Phenanthrolin; Jodacetamid; p-Hydroxymercuribenzoat; N-Ethylmaleimid; Phenylmethylsulfonylfluorid; Dithiothreitol und Glutathion auf die Aktivität; und
- zusätzlich befähigt zur Reduktion von p-Brom-acetophenon, Methylcyclohexanon, Aceton, Methylhexylketon 4-Phenyl-2-butanon, 1-Phenyl-1,2-propandion, Ethylpentylketon, Pinakolin, Propiophenon sowie p-Chlor-acetophenon.

Optisch aktiver Phenylethanol ist ein wertvoller chiraler Synthese-Baustein, der mit konventionellen Methoden schwer zugänglich ist.

Ein fermentatives Verfahren zur Herstellung von S(-)-Phenylethanol mit ruhenden Hefezellen wurde kürzlich von F. Aragozzini et al (Appl. Microbiol. Biotechnol. (1986) 24, 175-177) beschrieben: Danach wird S(-)-Phenylethanol aus Acetophenon (200 mg/l) bei 48 Std. Inkubation mit Hansenule glucozyma in 52%iger Ausbeute (92 % ee) und mit Torulopsis castellii in 20%iger Ausbeute (95 % ee) gebildet. R(+)-Phenylethanol entsteht dagegen (in 48 h) mit Aspergillus oryzae und Ne taria gliocladioides in 11 bzw. 14%iger Ausbeute mit 37 bzw. 40%iger EE.

Eine weiteres mikrobielles Verfahren zur Reduktion von Acetophenon wird in der JP-Appl. No. 60-128 289 vom 14.06.1985 (JP 61-285 993-A) beschrieben, das mit Bakterien vom Typ Pseudomonas durchgeführt wird und zu Zwischenprodukten zur Bildung von Arzneimitteln und Pestiziden führt. Angaben über die Stereospezifität der Reaktion sind nicht erkennbar.

Enzymkatalysierte Syntheseverfahren sind nicht bekannt. In der Literatur ist häufiger der Versuch beschrieben, mit kommerziell erhältlichen Alkohol-Dehydrogenasen, beispielsweise aus Hefe, Pferdeleber oder Thermoanaerobium brockii, Acetophenon zu reduzieren, diese Enzyme setzen Acetophenon aber nicht um (s. beispielsweise Keinan, E. et al (1987) Ann. N.Y. Acad. Sci. 501, 130 - 149, Tab. 5).

Es wurde nun überraschenderweise aus Lactobacillen eine Dehydrogenase isoliert, die in der Lage ist, R(+)-Phenylethanol aus der Ketokomponente (Acetophenon) herzustellen.

Besonders zweckmäßig wird dieses Enzym aus Lactobacillus kefir erhalten und insbesondere aus dem Lactobacillus kefir Stamm DSM 20 587.

Das Enzym benötigt NAOPH als Coenzym. Das Gleichgewicht der Reaktion liegt weit auf der Seite des Alkohols, so daß - beispielsweise bei kontinuierlicher Reaktionsführung unter Regenerierung des Coenzyms - das Enzym zur Synthese vonR(+)-Phenylethanol bzw. anderen sek. Alkoholen gut geeignet ist. Dabei läuft z.B. folgende Reaktion ab:

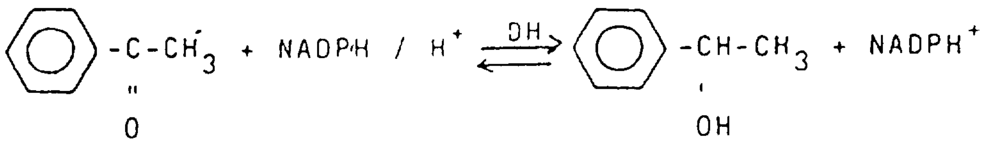


Acetophenon R(+)-Phenylethanol Die Erfindung umfaßt mithin ein Verfahren zur enzymatischen Reduktion von Oxo-Verbindungen unter Bildung optisch aktiver Hydroxy-Verbindungen, das dadurch gekennzeichnet ist, daß man die Oxo-Verbindung in Gegenwart von NAOPH mit Hilfe von erfindungsgemäßer Phenylethanol-Dehydrogenase umsetzt.

Vorzugsweise erfolgt dabei gleichzeitig eine NADPH-Regenerierung innerhalb der Reaktionsmischung, insbesondere mittels Glucose-6-P/Glucose-6-P-Dehydrogenase.

Die stereospezifische enzymatische Reaktion kann auch zur Gewinnung von S(-)-Alkohol eingesetzt werden, indem der R(+)-Alkohol aus einem raceinischen (R,S)-Alkoholgemisch in Gegenwart von $NADP^+$ enzymatisch mittels der Phenylethanol-Dehydrogenase zum Keton umgesetzt wird, das ohne Schwierigkeit vom verbleibenden S(-)-Alkohol abgetrennt werden kann. Vorzugsweise wird die enzymatische Reaktion in Gegenwart eines Ketons zur Regenerierung den $NADP^+$ durengeführt, das leicht abtrennbar ist und in großem Überschuß angewandt wird und für welches das Enzym einen möglichst kleinen $K_M$-Wert hat.

Nachfolgend wird die Erfindung mehr im einzelnen anhand spezieller Beispiele beschrieben, die mit dem Lactobacillus kefir Stamm DSM 20 587 durchgeführt wurden. Dabei wird auf die angefügten Kurvenbilder Bezug genommen; es zeigen:

Figur 1 die Bildung von Phenylethanol-Dehydrogenase bei der Fermentation von Lactabacillus kefir in Abhängigkeit von der Zeit;

Figur 2 die pH-Abhängigkeit der Aktivität der Phenylethanol-Dehydrogenase;

Figur 3 die Acetophenon-Abnahme bzw. Phenylethanol-Zunahme bei einer Batch-Umsetzung und

Figur 4 die chirale Trennung von R(+)- und S(-)-Phenylethanol mit

(a) racemischem Gemisch und

(b) dem enzymatischen Umsetzungsprodukt von Acetophenon.

(Retentionszeiten:

3,8 min : Derivatisierungsreagenz
10,4 min : R(+)-
11,7 min : S(-)-Phenylethanol
16,1 min : Acetophenon)

Beispiel 1: Gewinnung von Phenylethanol-Dehydrogenase

A. Züchtung von Lactobacillus kefir

Zur Enzymgewinnung wurde Lactobacillus kefir in folgendem Medium angezogen (pro 1 1):

| | |
|---|---|
| Glucose | 20 g |
| Hefeextrakt | 5 g |
| Universalpepton | 10 g |
| Fleischextrakt | 5 g |
| di-Ammoniumhydrogencitrat | 2 g |
| Natriumacetat | 5 g |
| Magnesiumsulfat | 0,1 g |
| Mangansulfat | 0,05 g |
| di-Kaliumhydrogenphosphat | 2 g |
| dest. $H_2O$ | 1 l |

Der pH-Wert dieser Lösung wurde auf 6,5 eingestellt, dann wurde für 15 min bei 121° C (2 bar) sterilisiert. Der Organismus wurde anaerob kultiviert, dazu reichte es aus, wenn das Medium mit $N_2$ überlagert wurde. Im 10 l-Maßstab wurde das Medium nach Erreichen der Bruttemperatur von 30° C mit 300 ml einer 24 Std alten Vorkultur beimpft. In einem solchen 10 l-Ansatz wurde beispielhaft der Verlauf der Enzymaktivität über der Zeit bestimmt, indem Proben zu verschiedenen Zeiten entnommen wurden, und die Aktivität der Phenylethanol-Dehydrogenase nach Aufschluß der Zellen bestimmt wurde. In Fig. 1 ist ein solcher Verlauf dargestellt, die Aktivität der Phenylethanol-Dehydrogenase erreicht nach kurzer Zeit einen maximalen Wert, der dann längere Zeit gehalten wird.

Im 70 l-Maßstab wurde der Organismus bei Raumtemperatur kultiviert, nach 75 Stunden wurde bei einem pH-Wert von 4,15 und einer $OD_{660}$ von 4,12 durch Separieren 320 g feuchte Zellmasse geerntet. Die Zellmasse kann bei -20° C eingefroren gelagert werden, wobei über mehrere Monate ist kein Aktivitätsverlust erkennbar ist.

B. Enzymisolierung Rohextrakt

Die Enzymfreisetzung aus den Zellen kann durch an sich bekannte.Methoden (Ultraschall, Hochdruck-Homogenisation, Naßvermahlung u.a.) geschehen. Hier wurden die Zellen durch Naßvermahlung mit Glasperlen aufgeschlossen. Dazu wurde die Bakterienmasse (80 g) in 100 mM Tris-HCl-Puffer (pH 9,0) unter Zusatz von 0,1% 2-Mercaptoethanol suspendiert, so daß die Konzentration der Zellfeuchtmasse 40%ig war (Endvolumen 200 ml). Die Zellinhaltsstoffe wurden aus der gekühlten Suspension (4° C) durch einen mechanischen Aufschluß mit Hilfe einer Glasperlenmühle (Dyno-Mill$^R$, Fa. Bachofen) freigesetzt. Der 340 ml fassende Mahlbehälter wurde dazu mit Glasperlen (0,5 mm) gefüllt, so daß sich ein Schüttvolumen von 290 ml ergab (85%iger Füllgrad). Der Aufschluß wurde bei einer Rührwellendrehzahl von 2000 UpM durchgeführt. Der Kühlmantel und das Rührwellenlager wurden während des Laufes gekühlt. 80 g Bakterienfeuchtmasse ergaben 138 ml Rohextrakt mit einer Volumenaktivität von 35 U/ml und einem Proteingehalt von 11

mg/ml. Hieraus läßt sich errechnen, daß aus 100 L Fermenteransatz ca. 27.000 Einheiten Phenylethanol-Dehydrogenase gewonnen werden können (1 Enzymeinheit ist die Enzymmenge, die 1 µMol Substrat/min umsetzt).

C. Anreicherung des Enzyms

Das Enzym kann durch Ionenaustausch-Chromatographie und Gelfiltration gereinigt werden. Dabei hat sich ein Zusatz von $Mg^{2+}$-Ionen (als Chlorid, Sulfat, etc.) während der Reinigung als absolut notwendig erwiesen. Wirksam ist beispielsweise eine Konzentration von 0,1 mM $MgCl_2$. Ohne einen solchen Zusatz desaktiviert das Enzym im Verlauf der Reinigung vollständig, durch nachträglichen Zusatz kann dieses Enzym nicht reaktiviert werden.

1. Ionenaustausch-Chromatographie: 1 ml Rohextrakt (entspr. Beispiel 1B) wird auf eine Mono-Q-Säule (Anionen-Austauscher) aufgetragen (FPLC-Chromatographie-System der Fa. Pharmacia, Freiburg, BRD). Die Säule ist mit Puffer A equilibriert worden (0,05 M Kaliumphosphat-Puffer, pH 7,0 mit 0.1 mM $MgCl_2$). Nach ausgiebigem Waschen der Säule mit Pufrer A wird die Phenylethanol-Dehydrogenase mit einem linearen NaCl-Gradienten (0 - 1 M) eluiert, wobei das Enzym bei ca. 0,35 M NaCl eluiert wird. Die erzielte Anreicherung ist in Tab. 1 zusammengefaßt.

2. Gelfiltration: Die Fraktion der Mono-Q-Chromatographie mit der höchsten Aktivität wird weiter aufgereinigt durch Chromatographie an Superose 6 HR 10/30 (FPLC-System der Fa. Pharmacia, Freiburg, BRD). Zur Equilibrierung und Chromatographie ist Puffer A eingesetzt worden, die Chromatgraphie wurde mit einer Flußrate von 0,5 ml/min durchgeführt. Die Anreicherung ist in Tab. 1 zusammengefaßt.

Tab. 1:

| Anreicherung der Phenylethanol-Dehydrogenase | | | | |
|---|---|---|---|---|
| Reinigungsschritt | Gesamt-Aktiv. | Spezif. Aktiv. | Ausbeute | Anreicherung |
| | U | U/mg | % | -fach |
| Rohextrakt | 16.9 | 1.4 | 100 | 1 |
| Mono-Q (Anionen-AT) | 14.5 | 28.3 | 86 | 20 |
| Superose (Gel-F.) | 12.8 | 99.1 | 76 | 70 |

Beispiel 2: Charakterisierung der Phenylethanol-Dehydrogenase

A. pH-Abhängigkeit der Umsetzung

Die Abhängigkeit der Aktivität vom pH-Wert wurde bestimmt, indem Acetophenon (11 mM) in verschiedenen Puffern gelöst wurde, die Puffer waren jeweils 0,1 M und hatten unterschiedliche pH-Werte (4,0 - 10,0; s. Fig. 1). Zu 970 µl einer solchen Substratlösung wurden 20 µl NADPH (Stammlösung 8 mg/ml) und 10 µl Enzymlösung zugesetzt und die Aktivität bei 340 nm (30°C) gemessen. Fig. 2 zeigt die erhaltenen Aktivitätswerte in Abhängigkeit vom pH-Wert, das Optimum für die Acetophenon-Reduktion liegt bei pH 7,0.

In analoger Weise wurde das pH-Optimum für die Rückreaktion, die Phenylethanol-Oxidation, gemessen. (±)-Phenylethanol (22 mM) wurde in 0,1 M Puffern mit pH-Werten zwischen 4,0 und 10,0 gelöst, 20 µl $NADP^+$ (165 mg/ml) und 10 µl Rohextrakt zugesetzt und die NADPH-Bildungsrate photometrisch gemessen. Fig. 2 faßt diese Werte zusammen, das pH-Optimum für die Oxidationsreaktion liegt bei 8,0.

B. Temperaturoptimum für die Acetophenon-Reduktion

Zur Bestimmung der optimalen Testtemperatur wurde die Enzymaktivität zwischen 25 und 40°C gemessen. Der Testansatz enthielt jeweils:

970 µl Acetophenon-Lösung (5,5 mM in Kaliumphosphat-Puffer,
pH 7,0; End-Konz. im Test: 5,3 mM);
20 µl NADPH (0,2 mM im Test)
10 µl Enzymlösung

Tab. 2 faßt diese Ergebnisse zusammen, das Enzym hat ein Temperaturoptimum bei 25-30°C, höhere Temperaturen

inaktivieren das Enzym.

Tab. 2:

| Abhängigkeit der Enzymaktivität von der Testtemperatur | |
|---|---|
| Temp. (°C) | Enzmyaktivität (U/ml) |
| 25 | 21,0 |
| 30 | 19,5 |
| 35 | 9,5 |
| 40 | 0 |

C. Einfluß verschiedener Metallkationen, Inhibitoren und SH-Schutzreagenzien

Für die folgenden Versuche wurde partiell gereinigtes Enzym (nach Mono-Q-Chromatographie, 20-fach angereichert) eingesetzt.

1. Metallkationen:

Der Einfluß von $Mg^{2+}$, $Zn^{2+}$ und $Mn^{2+}$-Ionen wurde untersucht, indem folgender Testansatz verwendet wurde:

0,1 M Kaliumphosphat-Puffer, pH 7,0
5,5 mM Acetophenon
0,2 mM NADPH
10 µl Enzym /ml (limitierende Konzentration: 1 - 10 µg Protein/ml),
$MgCl_2$, $ZnCl_2$ oder $MnCl_2$ wurden in einer Endkonz. von 1 mM zugesetzt.

Die Aktivität wurde bestimmt, indem die Änderung der NADPH-Konzentration durch Messung bei 340 nm pro 1 min gemessen wurde. Tab. 3 zeigt die stabilisierende Wirkung der Kationen, insbesondere diejenige von $Mg^{2+}$-Ionen.

2. Inhibitoren und SH-Schutzreagenzien:

Mit obigem Testansatz (Beispiel 2,C1) ohne Zusatz von Metallkationen wurden diverse Inhibitoren bzw. SH-Schutzreagenzien in 1 mM Endkonzentration dem Testansatz zugesetzt und die Restaktivität gemessen; p-Hydroxymercuribenzoat wurde wegen der Schwerlöslichkeit in 0,1 mM Konzentration eingesetzt.

Phenylmethansulfonylfluorid wurde in Acetonitril gelöst; die dadurch bedingte Acetonitril-Konzentration lag bei 1 mM und war, wie in Kontrollversuchen geprüft, ohne Einfluß auf die Enzymaktivität.

In Tab. 3 sind die Ergebnisse zusammengefaßt.

Auffallend ist, daß das Enzym durch den Chelator EDTA vollständig, durch die Chelatoren 1,10-Phenantrolin und 2,2-Dipyridin aber praktisch nicht gehemmt wird. Das spricht dafür, daß $Mg^{2+}$-Ionen notwendiger Bestandteil des Enzyms sind. Die nur schwache Hemmung durch die Inhibitoren Jodacetamid, p-Hydroxymercuribenzoat und N-Ethylmaleinimid zeigen, daß das Enzym anscheinend keine SH-Gruppe im aktiven Zentrum enthält. Das wird durch zugesetzte SH-Schutzreagenzien bestätigt, die hier keinen Einfluß zeigen.

Tab. 3:

| Einfluß von Metallkationen, Inhibitoren und SH-Schutzreagenzien | |
|---|---|
| Zusatz (%) | Restaktivität |
| *Metallkationen:* | |
| $MgCl_2$ | 132 |
| $ZnCl_2$ | 114 |
| $MnCl_2$ | 114 |
| | |
| *Inhibitoren und Chelatoren:* | |
| EDTA | 0 |

Fortsetzung der Tabelle auf der nächsten Seite

Tab. 3: (fortgesetzt)

| Einfluß von Metallkationen, Inhibitoren und SH-Schutzreagenzien | |
|---|---|
| Zusatz (%) | Restaktivität |
| 2,2-Dipyridine | 106 |
| 1,10-Phenanthroline | 99 |
| Jodacetamid | 90 |
| p-Hydroxymercuribenzoat | 92 |
| N-Ethylmaleinimid | 81 |
| Phenylmethylsulfonylfluorid (PMSF) | 94 |
| Triton X-100 | 94 |
| *SH-Schutz-Reagenzien:* | |
| Dithiothreitol | 95 |
| Glutathione | 99 |

D.h., übliche Inhibitoren, Chelatoren und SH-Schutzreagenzien haben nur einen geringen Einfluß auf die Aktivität des Enzyms

D. Abhängigkeit der Reaktionsgeschwindigkeit von der Acetophenonkonzentration

Zur Bestimmung der für die Umsetzung optimalen Acetophenon-Konzentration wurde die Enzymaktivität in Abhängigkeit von der Substratkonzentration gemessen. Folgender Testansatz wurde dazu eingesetzt:

970 µl Acetophenon-Lösung (in Kaliumphosphat-Puffer,
pH 7,0) var. Konz. zwischen 50 und 7100 µM;
20 µl NADPH (8,0 mg/ml Stammlösung; Konz. im Test: 0,2 mM)
10 µl Enzymlösung

Aktivitätsmessung photometrisch bei 340 nm, 30°C. Die Werte wurden jeweils um die substratunabhängige Hintergrund-reaktion (Testansatz mit 970 µl Puffer statt Acetophenon-Lösung) korrigiert. Die maximale Aktivität erreicht man bei Acetophenon-Konzentrationen von 5,3 mM (18,6 U/ml), der $K_M$-Wert liegt bei $6{,}0 \cdot 10^{-4}$ M Acetophenon. Bis zu einer Acetophenon-Konzentration von 10,6 mM tritt keine Substratüberschuß-Inhibierung auf.

E. Abhängigkeit der Reaktionsgeschwindigkeit von der NADPH-Konzentration

In Abhängigkeit von der NADPH-Konzentration wurde die Reaktionsgeschwindigkeit gemessen. Folgender Testansatz wurde verwendet:

970 µl Acetophenon-Lösung (5,5 mM in Kaliumphosphat-Puffer, pH 7,0; End-Konz. im Test: 5,3 mM);
20 µl NADPH (diverse Konz. zwischen 13 und 380 µM Endkonz.)
10 µl Enzymlösung

Aktivitätsmessung photometrisch bei 340 nm, 30°C. Maximale Aktivität konnte bei 190 µM NADPH gemessen werden (16,9 U/ml); der $K_M$-Wert beträgt $1{,}4 \cdot 10^{-4}$ M.

F. Substratspektrum der Phenylethanol-Dehydrogenase

Entsprechend Beispiel 2.D wurde an Stelle von Acetophenon eine Reihe aromatischer und langkettiger aliphatischer Ketone getestet, ob diese enzymkatalysiert reduziert werden können. Folgender Testansatz wurde dafür eingesetzt:

970 µl Keton-Lösung (in Kaliumphosphat-Puffer, pH 7,0) var. Konz. zwischen 10 µM und 10 mM je nach Akzeptans des jeweiligen Ketons;
20 µl NADPH (8,0 mg/ml Stammlösung;
Konz. im Test: 0,2 mM)
10 µl Enzymlösung (partiell gereinigt; 20-fach durch Mono-Q-Chromatographie)

In einigen wenigen Fällen wurde das Keton nur in einer Konzentration von 5 mM eingesetzt. Die Ergebnisse sind in Tabelle 4 zusammengestellt: Es zeigt sich, daß das Enzym eine Vielzahl aromatischer und aliphatischer sekundärer Ketone akzeptiert.

Tab. 4: Substratspezifität der Phenylethanol-Dehydrogenase.

1) n.b. = nicht bestimmt

| Substrate | $K_M$ (mM) | $v_{max}$ (%) |
|---|---|---|
| **A.) NADPH-abhängige Reduktionen:** | | |
| *Aromatische Verbindungen:* | | |
| Acetophenon (Methyl-phenylketon) | 0.600 | 100 |
| Propiophenon (Ethyl-phenylketon) | 2.74 | 43 |
| 4-Phenyl-2-butanon | 9.40 | 90 |
| 4-Bromo-acetophenon | 0.372 | 101 |
| 1-Phenyl-1,2-propandion | n.b.[1] | 70 |
| Methyl-2-naphthylketon | n.b. | 11 |
| Benzaldehyd | n.b. | 10 |
| 4-Chlor-acetophenon | 0.390 | 93 |
| *Zyklische und azyklische aliphatische Verb.:* | | |
| (±)-Methylcyclohexanon | 70.0 | 109 |
| Pinakolin (*tert.*-Butyl-methylketon) | 3.38 | 63 |
| Methyl-hexylketon | 0.251 | 91 |
| Ethyl-pentylketon | 0.681 | 66 |
| Mesityloxid (4-Methyl-3-penten-2-on) | n.b. | 12 |
| Aceton | 37.9 | 96 |
| NADPH | 0.14 | 100 |
| **B.) $NADP^+$-abhängige Oxidationen:** | | |
| R(+)-Phenylethanol | 3.5 | 11 |
| (±)-Phenylethanol | 4.3 | 17 |
| Isopropanol | 0.117 | 18 |
| $NADP^+$ | 0.19 | 17 |

Das Enzym ist mithin zur Reduktion von einer Reihe von aromatischen, alicyclischen und aliphatischen Ketonen befähigt.

Beispiel 3: Enzymkatalysierte Herstellung von (+)-Phenylethanol im batch

Eine 10 mM Lösung von Acetophenon wurde mit Enzym (0,5 U/ml) und Coenzym (0,05 mM) umgesetzt (5 ml gesamt), dabei wurde das Coenzym NADPH durch Kopplung mit Glucose-6-phosphat (15 mM) und Glucose-6-phosphat-Dehydrogenase (0,5 U/ml) regeneriert. Im Abstand von 10 min wurden Proben entnommen (50 µl) und mit Hilfe der HPLC auf den Gehalt an Phenylethanol und Acetophenon analysiert.

Dazu wurden die Proben jeweils mit 50 µl Aceton versetzt, das ausgefallene Protein abzentrifugiert und 50 µl des

Überstands mit 450 µl HPLC-Laufmittel versetzt.

Trennbedingungen der HPLC:

Säule: ODS-Hypersil 5µ (250x4,6 mm); Laufmittel: Tris-HCl pH 8,4 mit 35% Acetonitril (filtriert und mit Helium begast); Flußrate: 1 ml/min; Temperatur: 25°C (Kühlofen); Detektion: photometrisch bei 226 nm; Probenmenge: 40 µl; Elutionszeiten: Acetophenon = 12,6 min, Phenylethanol = 7,8 min.

Fig. 3 zeigt, daß im Verlauf der Reaktion Acetophenon von anfänglich 10 mM kontinuierlich bis auf ca. 0,5 mM abnimmt und symmetrisch dazu Phenylethanol gebildet wird.

In zwei weiteren Experimenten wurden andere Regenerierungsmöglichkeiten für das Coenzym NADPH geprüft. In obigem Ansatz wurden Glucose-6-phosphat und Glucose-6-phosphat-Dehydrogenase einerseits durch Glucose (15 mM) und Glucose-Dehydrogenase (0,5 U/ml) ersetzt, andererseits durch Isopropanol (20mM). Isopropanol ist auch Substrat der Phenylethanol-Dehydrogenase, unter Reduktion von $NADP^+$ wird dabei stöchiometrisch Aceton gebildet. Wie in obigem Versuch wurden alle 10 min Proben entnommen und der Gehalt an Phenylethanol mittels HPLC bestimmt. In Tabelle 5 sind repräsentative Werte dieser drei Regenerierungsmöglichkeiten zusammengestellt.

Tab. 5:

| Umsetzung von 10 mM Acetophenon in Gegenwart verschiedener NADPH-Regenerierungsmittel (DH = Dehydrogenase) | | | |
|---|---|---|---|
| NADPH Regenerierung durch | Phenylethanol (mM) nach | | |
| | 10 min | 30 min | 60 min |
| Glucose/Glucose-DH | 1.8 | 3.8 | 7.9 |
| Glucose-6-P/Glucose-6-P-DH | 6.2 | 8.8 | 8.6 |
| Isopropanol | 1.6 | 4.6 | 8.0 |

Beispiel 4: Enzymkatalysierte Herstellung von (+)-Phenylethanol im Enzym-Membran-Reaktor

Eine kontinuierliche Synthese von Phenylethanol ist möglich unter Verwendung eines Enzym-Membran-Reaktors. Hier werden Enzyme durch eine Ultrafiltrationsmembran (YM 5, Fa. Amicon, Witten, BRD) im Reaktor (CEC 1, Fa. Amicon) zurückgehalten, während die niedermolekularen Bestandteile der Reaktionslösung (nichtumgesetztes Substrat, Produkt, Puffer) laufend aus der Lösung entfernt werden (Verweilszeit 2 Std.). Das Reaktorvolumen wird konstant gehalten, indem in gleichem Maß aus einem Reservoir 10 mM Acetophenon in Puffer ( 0,1 M Kaliumphosphat, pH 7,0) nachdosiert wird, wie das Ultrafiltrat den Reaktor verläßt. Das Reaktorvolumen betrug 10 ml, es enthielt im einzelnen: 0,5 U/ml Phenylethanol-Dehydrogenase, 0,5 U/ml Glucose-6-phosphat-Dehydrogenase, 5 mM Glucose-6-phosphat, 10 mM Acetophenon, 0,4 mM $NADP^+$, in 0,1 M Kaliumphosphat-Puffer pH 7,0. Im Abstand von 4 Std. wurden $NADP^+$ und Glucose-6-phosphat in das Reaktionsgefäß nachdosiert, so daß obige Endkonzentrationen erreicht wurden.

Die Umsetzung wurde analytisch verfolgt, indem der Gehalt an Acetophenon und Phenylethanol in Proben (ca. alle 3 Std.) mit Hilfe einer HPLC wie in Beispiel 3 beschrieben gemessen wird. Das Ergebnis ist in Tab. 6 zusammengefaßt.

Tab. 6:

| Kontinuierliche Umsetzung von Acetophenon zu Phenylethanol im Enzym-Membran-Reaktor | | |
|---|---|---|
| Zeit (Std.) | Acetophenon (mM) | Phenylethanol (mM) |
| 0 | 10 | 0 |
| 1 | 3,8 | 5,2 |
| 4 | 2 | 6,9 |
| 8 | 0,8 | 7,8 |
| 26 | 0,6 | 7,7 |
| 32 | 0,2 | 8,7 |
| 46 | 0,2 | 8,6 |
| 52 | 0,2 | 8,4 |

Beispiel 5: Nachweis der Stereospezifität des Enzyms

Zum Nachweis der Stereospezifität des Enzyms und der Enantiomerenreinheit des Produkts wurden zwei Methoden verwendet, einerseits wurde enzymatisch hergestelltes Produkt mit einer chiralen HPLC, die R(+)- und S(-)-Phenylethanol trennen kann, untersucht, amdererseits wurde mit käuflich erhältlichen, reinen Isomeren von R(+)- bzw. S(-)-Phenylethanol photometrisch die Oxidationsreaktion gemessen.

A. Chirale HPLC

Um größere Mengen an enzymatisch hergestelltem Produkt für die Analytik einsetzen zu können, wurde das Produkt der kontinuierlichen Umsetzung von Acetophenon verwendet, das unter Regenerierung des Coenzyms NADPH hergestellt worden ist. Die Umsetzung selbst ist in Beispiel 4 ausführlich beschrieben.

Eine Trennung der beiden Enantiomeren ist unter den gewählten Bedingungen nur nach Derivatisierung des Phenylethanols zum Benzoyl-Derivat möglich.

Dazu wurde 1 ml der Produktlösung mit 1 ml Dichlormethan ausgeschüttelt, nach Abtrennung der wässrigen Phase das Dichlormethan entfernt und dann 1 ml Pyridin zugesetzt. Zur Derivatisierung wurden 5 µl Benzoylchlorid zugesetzt und 30 min bei Raumtemperatur stehengelassen. Nach Zusatz von 0,5 ml $H_2O$ wurde das Produkt mit 2 ml Hexan extrahiert und mittels HPLC analysiert.

Trennbedingungen: Säule: Nucleosil-Chiral 2 (250x4 mm) der Fa. Macherey und Nagel (Düren, FRG); Laufmittel: n-Heptan mit 0,05% 1-Propanol und 0,05% Trifluoressigsäure (Restwasser wurde mit einem Molekularsieb 0,4 Å entfernt); Temperatur: 25°C (mittels Kühlofen); Flußrate: 1 ml/min; Detektion spektrophotometrisch bei 250 nm; Probenschleife: 40µl. Die Zuordnung des Produkt-Peaks bei 10,4 min geschah, indem die Derivatisierung mit käuflich erhältlichen reinen Verbindungen von R(+)- und S(-)-Phenylethanol (Fa. Fluka) durchgeführt wurde.

Fig. 4 zeigt, daß man durch enzymatische Reduktion reines R(+)-Phenylethanol erhält, S(-)-Phenylethanol ist nicht nachweisbar.

B. Rückreaktion mit R(+)- bzw. S(-)-Phenylethanol

Zum photometrischen Nachweis der Stereospezifität wurden folgende Testmischungen eingesetzt:

I) 970 µl R(+)-Phenylethanol (11 mM; in Kaliumphospatpuffer 0,1 M, pH 8,0)
20 µl $NADP^+$ (165 mg/ml)
10 µl Enzymlösung

II) 970 µl S(-)-Phenylethanol (11 mM; in Kaliumphospatpuffer 0,1 M, pH 8,0)
20 µl $NADP^+$ (165 mg/ml)
10 µl Enzymlösung

Zum Vergleich wurde ein dritter Ansatz mit 22 mM (±)-Phenylethanol eingesetzt.

Die Aktivitätsmessung geschah photometrisch bei 340 nm (30°C).

Es wurde gemessen:

| | |
|---|---|
| Ansatz I: | 3,5 U/ml |
| Ansatz II: | 0 |
| Ansatz III: | 2,3 U/ml |

Das Ergebnis zeigt eindeutig, daß das Enzym hochspezifisch für R(+)-Phenylethanol ist, der Vergleich mit Ansatz III zeigt, daß die gleichzeitige Anwesenheit von S(-)-Phenylethanol das Enzym sogar hemmt.

Beispiel 6: Präparation von S(-)-Alkoholen

Die Herstellung von S(-)Alkoholen mit Hilfe des Enzyms aus Lactobacillus kefir ist möglich, indem das racemische Gemisch des Alkohols, z.B. (R,S)-Phenylethanol, als Substrat eingesetzt wird und enzymatisch die R-Komponente zum entsprechenden Keton oxidiert wird. Vorteilhaft ist dabei der Zusatz eines Ketons, das selbst Substrat des Enzyms ist, beispielsweise Aceton, sodaß das sich bildende NADPH wieder oxidiert werden kann und die Reaktion dadurch begünstigt ablaufen kann. Das Coenzym wird dann nur noch in katalytisch gerade noch wirksamen Mengen benötigt. Am Beispiel der Herstellung von S-Phenylethanol ist das in den Gleichungen (1)-(3) zusamniengestellt.

ENZYM
(1) R,S-Phenylethanol + $NADP^+$ ---> Acetophenon + S-Phenylethanol + NADPH + $H^+$

ENZYM
(2) Aceton + NADPH + $H^+$ ----> Isopropanol + $NADP^+$

ENZYM,$NADP^+$
(3) R,S-Phenylethanol + Aceton -----> Acetophenon + Isopropanol + S-Phenylethanol

Zur Herstellung von S(-)-Phenylethanol wurden folgende Komponenten entspr. der Tabelle eingesetzt:

Tab. 6:

| Bedingungen für die enzymatische Präparation von S-Phenylethanol aus R,S-Phenylethanol | | |
|---|---|---|
| Bestandteil | Reaktion A (Endkonzentration) | Reaktion B |
| R,S-Phenylethanol | 5 mM | 5 mM |
| Kaliumphosphat-Puffer (0,4 M; pH 7,0) | 875 µl | 875 µl |
| $MgCl_2$ x 6 $H_2O$ | 0,1 mM | 0,1 mM |
| $NADP^+$ | 1 mM | 1 mM |
| Phenylethanol-Dehydrogenase (28 U/mg) | 0,6 U | 0,6 U |
| Aceton | ---- | 10 mM |

Die Reaktion läuft bei 30°C ab, zu den Zeitpunkten 0,10, und 30 min werden Proben entnommen und der Gehalt an Phenylethanol und Acetophenon, wie in Beispiel 3 beschrieben, mittels HPLC bestimmt. Die Ergebnisse sind in Tab. 7 zusammengestellt.

Tab. 7:

| Enzymatische Umsetzung von R,S-Phenylethanol (5 mM) | | | |
|---|---|---|---|
| Ansatz | Reaktionszeit (min) | Acetophenon (mM) | Phenylethanol (mM) |
| A | 0 | 0 | 5 |
| A | 10 | 0,21 | 4,79 |
| A | 30 | 0,38 | 4,62 |
| B | 0 | 0 | 0 |
| B | 10 | 0,50 | 4,50 |
| B | 30 | 0,88 | 4,12 |

**Patentansprüche**

1. Zur Reduktion von Acetophenon zu praktisch enantiomeren-reinem R(+)-Phenylethanol in Gegenwart von NADPH befähigte, aus Lactobacillen isolierbare Phenylethanol-Dehydrogenase mit folgendem weiteren Reaktionsspektrum

   - rasche Desaktivierung durch EDTA, aber geringer Einfluß von 2,2-Dipyridin; l,10-Phenanthrolin; Jodacetamid; p-Hydroxymercuribenzoat; N-Ethylmaleimid; Phenylmethylsulfonylfluorid; Dithiothreitol und Glutathion auf die Aktivität; und

   - zusätzlich befähigt zur Reduktion von p-Brom-acetophenon, Methylcyclohexanon, Aceton, Methylhexylketon 4-Phenyl-2-butanon, 1-Phenyl-1,2-propandion, Ethylpentylketon, Pinakolin, Propiophenon sowie p-Chlor-acetophenon.

2. Dehydrogenase nach Anspruch 1, isoliert aus Lactobacillus kefir.

3. Dehydrogenase nach Anspruch 2, isoliert aus dem Lactobacillus kefir Stamm DSM 20 587.

4. Verfahren zur enzymatischen Reduktion von Keto-Verbindungen unter Bildung optisch aktiver Hydroxyverbindungen,
**dadurch gekennzeichnet**,
daß man die Keto-Verbindung in Gegenwart von NADPH mit Hilfe von Phenylethanol-Dehydrogenase nach einem der Ansprüche 1 - 3 umsetzt.

5. Verfahren nach Anspruch 4,
**gekennzeichnet durch**
die gleichzeitige NADPH-Regenerierung innerhalb der Reaktionsmischung, insbesondere mittels Glucose-6-P/Glucose-G-P-Dehydrogennse.

6. Verfahren zur Gewinnung von S(-)-Phenylethanol aus einem racemischen Gemisch des (R,S)-Alkohols durch Abtrennung des (R)-Alkohols,
**dadurch gekennzeichnet**,
daß man das racemische Gemisch mit der Phenylethanol-Dehydrogenase nach einem der Ansprüche 1 bis 3 in Gegenwart von $NADP^+$ enzymatisch umsetzt und das gebildete Keton vom nicht umgesetzten S(-)-Phenylethanol abtrennt.

7. Verfahren nach Anspruch 6,
**dadurch gekennzeichnet**,
daß man die enzymatische Umsetzung in Gegenwart von Keton zur Regenerierung von $NADP^+$ durchführt.

**Claims**

1. Phenylethanol dehydrogenase isolable from lactobacilli and capable of reducing acetophenone to virtually enantiomerically pure R(+)-phenylethanol in the presence of NADPH, having the following additional reaction spectrum

   - rapid deactivation by EDTA, but marginal effect of 2,2-dipyridine; 1,10-phenanthroline; iodoacetamide; p-hydroxymercurybenzoate; N-ethylmaleimide; phenylmethylsulfonyl fluoride; dithiothreitol and glutathione on the activity; and

   - additionally capable of reducing p-bromoacetophenone, methylcyclohexanone, acetone, methyl hexyl ketone, 4-phenyl-2-butanone, 1-phenyl-1,2-propanedione, ethyl-pentyl ketone, pinacolone, propiophenone and p-chloroacetophenone.

2. Dehydrogenase according to Claim 1, isolated from Lactobacillus kefir.

3. Dehydrogenase according to Claim 2, isolated from the Lactobacillus kefir strain DSM 20 587.

4. Process for the enzymatic reduction of keto compounds with formation of optically active hydroxy compounds, characterized in that the keto compound is reacted in the presence of NADPH using phenylethanol dehydrogenase according to one of Claims 1-3.

5. Process according to Claim 4, characterized by the simultaneous regeneration of NADPH within the reaction mixture, in particular by means of glucose-6-P/glucose-6-P-dehydrogenase.

6. Process for obtaining S(-)-phenylethanol from a racemic mixture of the (R,S)-alcohol by separating off the (R)-alcohol, characterized in that the racemic mixture is enzymatically reacted in the presence of $NADP^+$ with the phenylethanol dehydrogenase according to one of Claims 1 to 3 and the ketone formed is separated off from the unreacted S(-)-phenylethanol.

7. Process according to Claim 6, characterized in that the enzymatic reaction is carried out in the presence of ketone for the regeneration of $NADP^+$.

## Revendications

1. Phényléthanol-déshydrogénase capable de provoquer la réduction de l'acétophénone en énantiomère pratiquement pur R-(+)-phényléthanol, en présence de NADPH, pouvant être isolée des lactobacilles et ayant, en outre, le spectre suivant de réaction :

   - désactivation rapide par EDTA, mais faible influence de la 2,2-dipyridine, de la 1,10-phénanthroline, de l'iodoacétamide, du p-hydroxymercuribenzoate, du N-éthylmaléimide, du fluorure de phénylméthylsulfonyle, du dithiothréitol et du glutathion sur l'activité ; et
   - en outre, capable de provoquer la réduction de la p-bromoacétophénone, de la méthylcyclohexanone, de l'acétone, de la méthylhexylcétone, de la 4-phényl-2-butanone, de la 1-phényl-1,2-propanedione, de l'éthylpentylcétone, de la pinacolone, de la propiophénone, ainsi que de la p-chloroacétophénone.

2. Déshydrogénase selon la revendication 1, isolée de *Lactobacillus kefir.*

3. Déshydrogénase selon la revendication 2, isolée de la souche DSM 20 587 de *Lactobacillus kefir.*

4. Procédé de réduction enzymatique de composés céto avec formation de composés hydroxy optiquement actifs, caractérisé en ce qu'on fait réagir les composés céto en présence de NADPH à l'aide d'une phényléthanol-déshydrogénase selon l'une des revendications 1 à 3.

5. Procédé selon la revendication 4, caractérisé par la régénération simultanée de NADPH dans le mélange de réaction, en particulier au moyen de glucose-6-P/glucose-6-P-déshydrogénase.

6. Procédé d'obtention de S-(-)-phényléthanol à partir d'un mélange racémique de l'alcool (R,S), par séparation de l'alcool (R), caractérisé en ce qu'on fait réagir par voie enzymatique le mélange racémique avec la phényléthanol-déshydrogénase selon l'une des revendications 1 à 3 en présence de $NADP^+$ et qu'on sépare la cétone formée d'avec le S(-)-phényléthanol n'ayant pas subi de réaction.

7. Procédé selon la revendication 6, caractérisé en ce qu'on effectue la réaction enzymatique en présence d'une cétone pour la régénération de $NADP^+$.

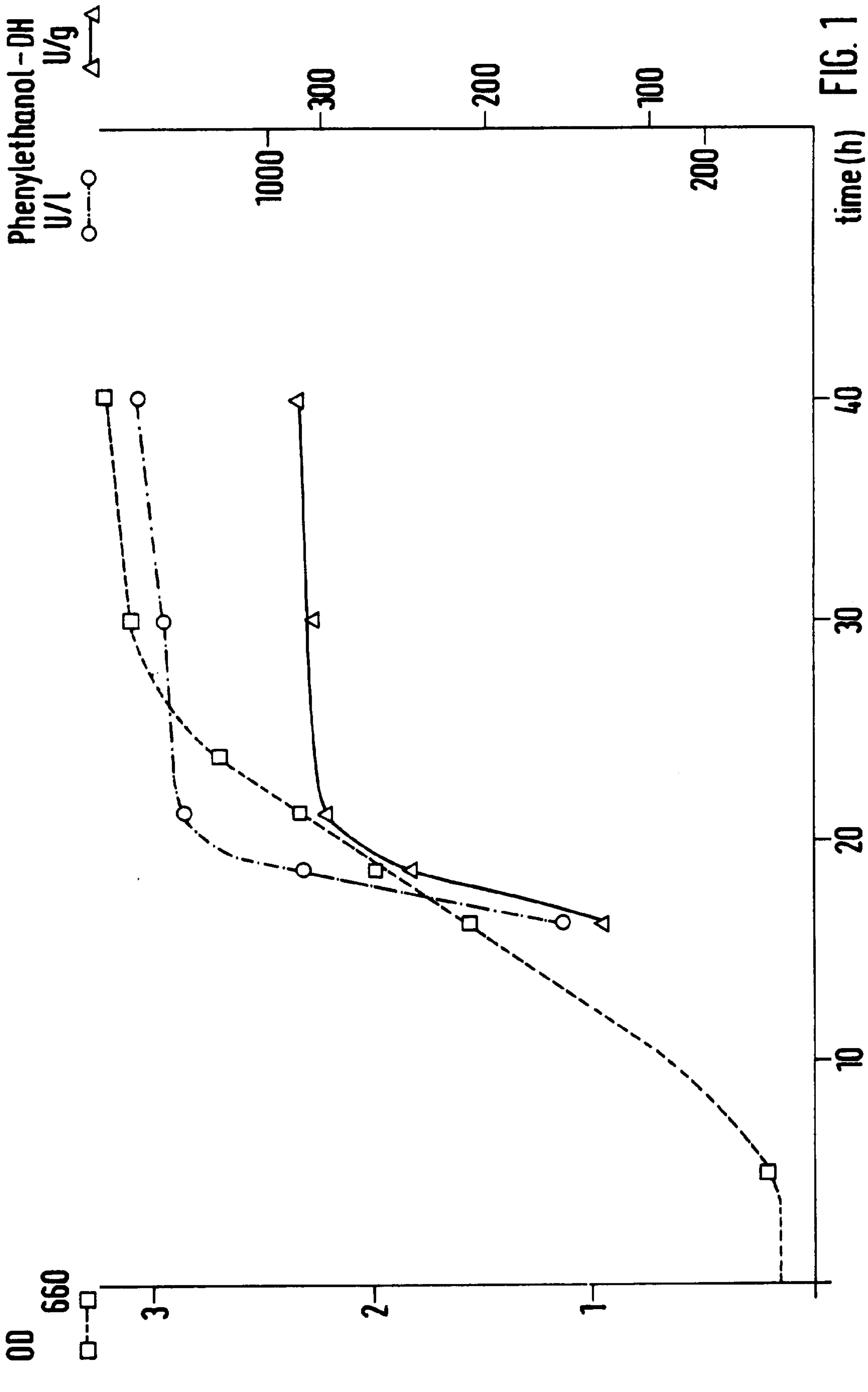

OD 660
3
2
1
Phenylethanol-DH
U/l
U/g
1000
200
300
200
100
time (h)
10
20
30
40


FIG. 1

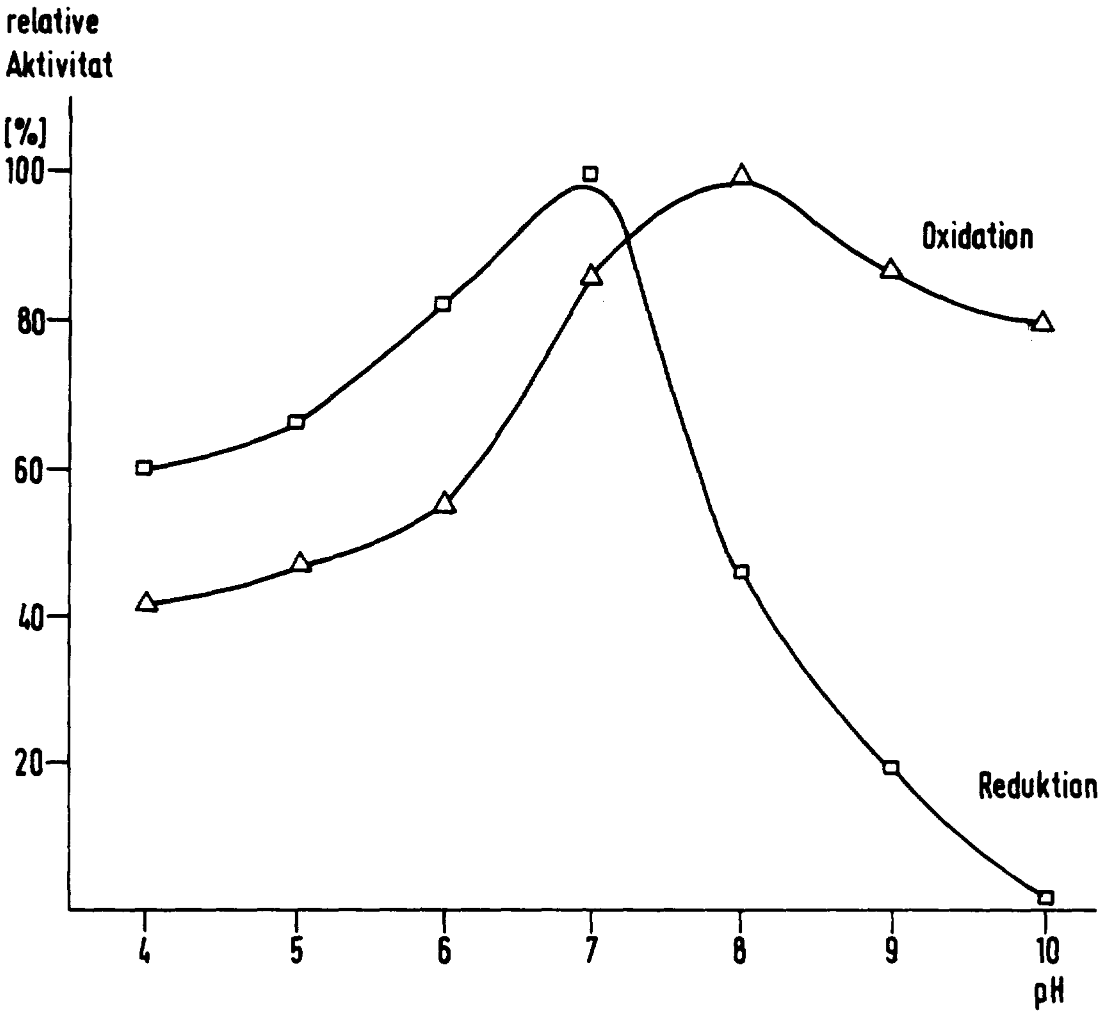
relative
Aktivitat
[%]
100
80
60
40
20
4
5
6
7
8
9
10
pH
Oxidation
Reduktion

FIG. 2

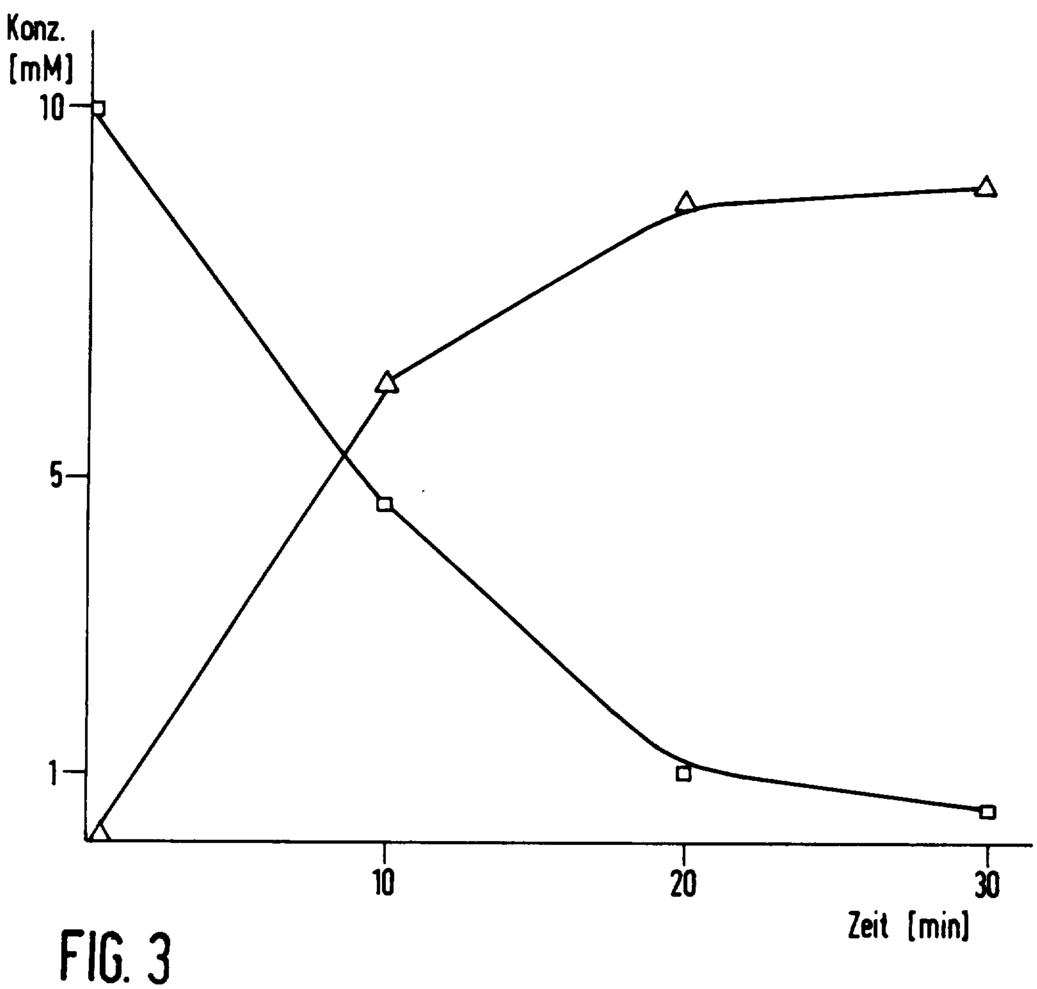


FIG. 3

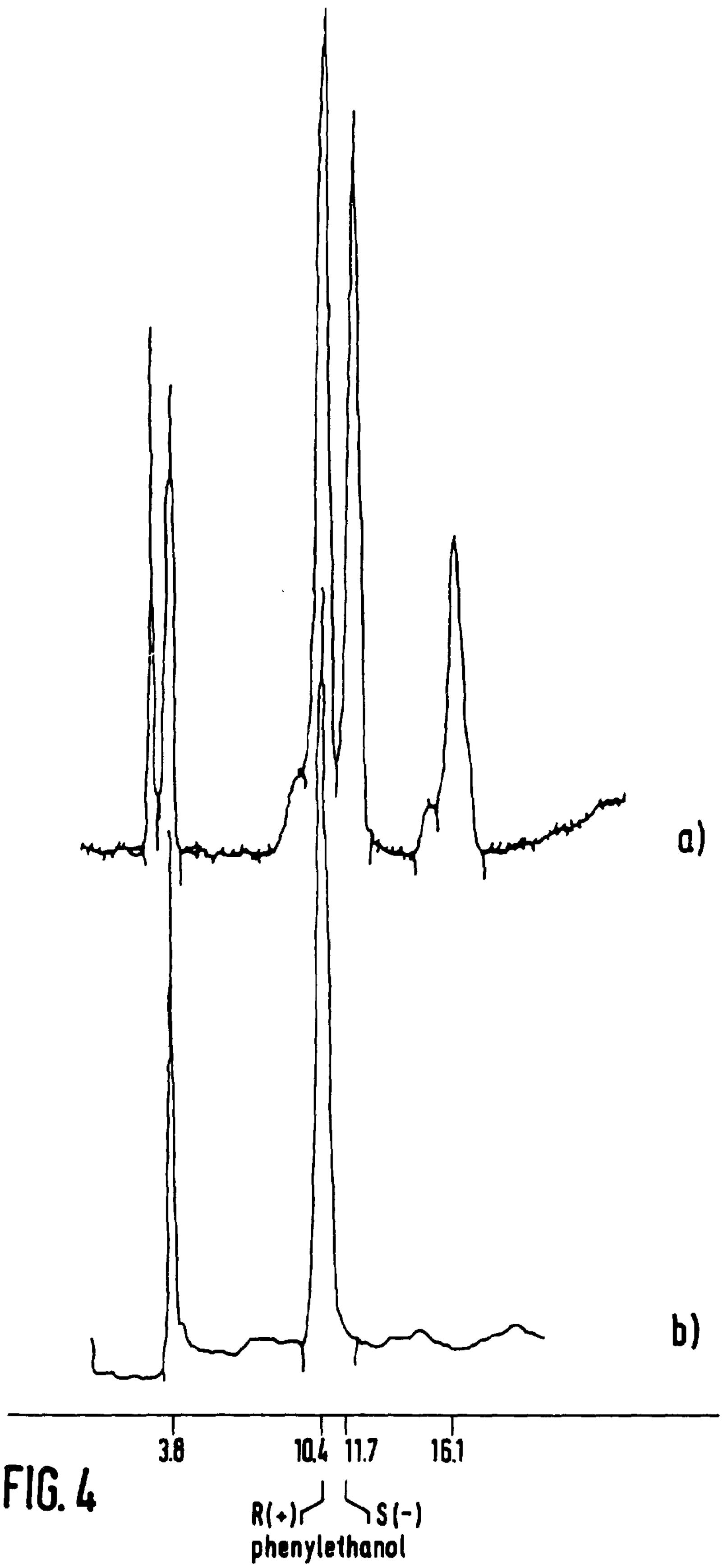


FIG. 4